# FASCICULE DE BREVET EUROPEEN

(11) **EP 0 833 818 B1**
(45) Date de publication et mention de la délivrance du brevet: **17.10.2001**
(21) Numéro de dépôt: 96922962.4
(22) Date de dépôt: 20.06.1996
(51) Int. Cl.: C07D 209/12, A61K 31/40, C07D 209/16, C07D 498/06, C07D 471/06, C07D 209/08

(54) **3-ACYLINDOLES COMME AGONISTES DU RECEPTEUR CB2**
3-ACYLINDOLE ALS CB2 REZEPTOR AGONISTEN
3-ACYLINDOLES AS CB2 RECEPTOR AGONISTS

(30) Priorité: 21.06.1995 FR 9507438
(43) Date de publication de la demande: 08.04.1998
(73) Titulaire: SANOFI-SYNTHELABO, 75013 Paris (FR)
(72) Inventeur: RINALDI, Murielle, F-34680 Saint-Georges-d'Orques (FR); BARTH, Francis, F-34070 Montpellier (FR); CASELLAS, Pierre, F-34000 Montpellier (FR); CONGY, Christian, F-34980 Saint-Gely-du-Fesc (FR); OUSTRIC, Didier, F-34920 Le Cres (FR); BELL, Malcolm, R., East Greenbusch, NY 12061 (US); D'AMBRA, Thomas, E., Wynantskill, NY 12198 (US); PHILION, Richard, E., Pottstown, PA 19464 (US)
(74) Mandataire: Gillard, Marie-Louise
(86) Numéro de dépôt international: FR9600959
(87) Numéro de publication internationale: WO9700860

(56) Documents cités:
- EP-A- 0 444 451
- US-A- 4 973 587
- US-A- 5 013 837
- US-A- 5 081 122
- CHEMICAL ABSTRACTS, vol. 120, no. 7, 14 Février 1994 Columbus, Ohio, US; abstract no. 71070e, KAMINSKI,N.E.: "Evidence for a cannabinoid receptor in immunomodulation by cannabinoid compounds" XP002014386 & ADV. EXP. MED. BIOL., vol. 335, - 1993 pages 115-120,
- JOURNAL OF MEDICINAL CHEMISTRY, vol. 35, no. 1, - 1992 WASHINGTON US, pages 124-135, XP002014381 THOMAS E.D'AMBRA ET AL.: "Conformationally restrained analogues of pravadoline: ..."
- CHEMICAL ABSTRACTS, vol. 99, no. 25, 19 Décembre 1983 Columbus, Ohio, US; abstract no. 212416j, SARBU,CONSTANTIN ET AL.: "2-Methyl-3-ethylindole derivatives" XP002014387 & RO,A,77 049 (INSTITUTUL DE CERCETARI CHIMICO-FARMACEUTICE)
- CHEMICAL ABSTRACTS, vol. 107, no. 15, 12 Octobre 1987 Columbus, Ohio, US; abstract no. 134196e, SARBU,CONSTANTIN ET AL.: "Preparation of 1-(p-chlorobenzoyl)-3-(2-(dipropylamino)et hyl)-5-methoxy-2-methylindole hydrochloride hydrate" XP002014388 & RO,A,90 049
- CHEMICAL ABSTRACTS, vol. 93, no. 11, 15 Septembre 1980 Columbus, Ohio, US; abstract no. 107063v, SARBU,C. ET AL.: "New indole derivatives ..." XP002014389 & REV. ROUM. CHIM., vol. 25, no. 2, - 1980 pages 245-251,
- CHEMICAL ABSTRACTS, vol. 121, no. 1, 4 Juillet 1994 Columbus, Ohio, US; abstract no. 228x, HUFFMANN,JOHN W. ET AL.: "Design,synthesis and pharmacology of cannabimimetic indoles" XP002014390 & BIOORG. MED. CHEM. LETT., vol. 4, no. 4, - 1994 pages 563-566,
- JOURNAL OF MEDICINAL CHEMISTRY, vol. 34, no. 3, - 1991 WASHINGTON US, pages 1099-1110, XP002014382 MALCOLM R. BELL ET AL.: "Antinociceptive (aminoalkyl)indoles"
- CHEMICAL ABSTRACTS, vol. 115, no. 5, 5 Août 1991 Columbus, Ohio, US; abstract no. 41785w, PACHECO,MARY ET AL.: "Aminoalkylindoles: action on specific G-protein-linked receptors" XP002014391 & J. PHARMACOL. EXP. THER. , vol. 257, no. 1, - 1991 pages 170-183,
- CHEMICAL ABSTRACTS, vol. 124, no. 17, 22 Avril 1996 Columbus, Ohio, US; abstract no. 232174s, D'AMBRA,THOMAS E. ET AL.: "C-attached aminoalkylindoles: potent cannabinoid mimetics" XP002014392 & BIOORG. MED. CHEM. LETT., vol. 6, no. 1, - 1996 pages 17-22,
- CHEMICAL ABSTRACTS, vol. 123, no. 3, 17 Juillet 1995 Columbus, Ohio, US; abstract no. 25542z, PERTWEE,ROGER ET AL.: "AM630, a competitive cannabinoid receptor antagonist" XP002014393 & LIFE SCI., vol. 56, no. 23/24, - 1995
- JOURNAL OF MEDICINAL CHEMISTRY, vol. 39, no. 10, - 10 Mai 1996 WASHINGTON US, pages 1967-1974, XP002014383 KOICHIRO YAMADA ET AL.: "(Aminoalkyl)indole isothiocyanates as ..."
- JOURNAL OF MEDICINAL CHEMISTRY, vol. 38, no. 16, - 4 Août 1995 WASHINGTON US, pages 3094-3105, XP002014384 MICHAEL A. EISSENSTAT ET AL.: "Aminoalkylindoles: structure-activity relationships of novel cannabinoid mimetics"
- FEBS LETTERS, vol. 369, - 7 Août 1995 pages 177-188, XP002014394 J.-M. DEROCQ ET AL.: "Cannabinoids enhance human B-cell growth at low nanomolar concentrations"

## Description

La présente invention a pour objet des nouveaux composés agonistes du récepteur CB₂ humain et les compositions pharmaceutiques les contenant ainsi que les procédés pour leur obtention et leur utilisation pour la préparation de médicaments immunomodulateurs.

Le Δ⁹-THC est le principal constituant actif extrait de *Cannabis sativa* (Tuner, 1985 ; In Marijuana 1984, Ed. Harvey, DY, IRL Press, Oxford).

De nombreux articles ont décrit non seulement des effets psychotropes des cannabinoïdes mais aussi une influence de ces derniers sur la fonction immunitaire [HOLLISTER L.E. J. Psychoact. Drugs 24 (1992), 159-164]. La plupart des études in vitro ont montré des effets immunosuppresseurs des cannabinoïdes : l'inhibition des réponses prolifératives des lymphocytes T et des lymphocytes B induites par les mitogènes [Luo, Y.D. et al., Int. J. Immunopharmacol. (1992) 14, 49-56, Schwartz, H. et al., J. Neuroimmunol. (1994) 55, 107-115], l'inhibition de l'activité des cellules T cytotoxiques [Klein et al., J. Toxicol. Environ. Health (1991) 32, 465-477], l'inhibition de l'activité microbicide des macrophages et de la synthèse du TNFα [Arata, S. et al., Life Sci. (1991) 49, 473-479 ; Fisher-Stenger et al. J. Pharm. Exp. Ther. (1993) 267, 1558-1565], l'inhibition de l'activité cytolytique et de la production de TNFα des grands lymphocytes granulaires [Kusher et al. Cell. Immun. (1994) 154, 99-108]. Dans certaines études, des effets d'amplification ont été observés : augmentation de la bioactivité de l'interleukine-1 par les macrophages résidant de souris ou les lignées cellulaires macrophagiques différenciées, due à des niveaux accrus de TNFα [Zhu et al., J. Pharm. Exp. Ther. (1994) 270, 1334-1339 ; Shivers, S.C. et al. Life Sci. (1994) 54, 1281-1289].

Les effets des cannabinoïdes sont dus à une interaction avec des récepteurs spécifiques de haute affinité présents au niveau central (Devane et al., Molecular Pharmacology (1988), 34, 605-613) et périphérique (Nye et al., The Journal of Pharmacology and Experimental Therapeutics (1985), 234, 784-791 ; Kaminski et al., Molecular Pharmacology (1992), 42, 736-742 ; Munro et al., Nature (1993), 365, 61-65).

Les effets centraux relèvent d'un premier type de récepteur de cannabinoïdes (CB₁) qui est présent dans le cerveau. Par ailleurs, Munro et al. [Nature (1993) 365, 61-65] ont cloné un second récepteur de cannabinoïdes couplé à la protéine G, appelé CB₂, qui est seulement présent à la périphérie et plus particulièrement sur les cellules d'origine immune. La présence de récepteurs aux cannabinoïdes CB₂ sur les cellules lymphoïdes peut expliquer l'immunomodulation exercée par les agonistes des récepteurs aux cannabinoïdes évoquée ci-dessus.

Les agonistes des récepteurs aux cannabinoïdes connus jusqu'à présent sont des agonistes mixtes, c'est-à-dire qu'ils agissent aussi bien sur les récepteurs centraux (CB₁) que sur les récepteurs périphériques (CB₂). Il en résulte que si l'on veut traiter le système immunitaire avec les agonistes connus des récepteurs aux cannabinoïdes, on a toujours un effet secondaire non négligeable, qui est l'effet psychotrope.

Les brevets suivants décrivent des agonistes non sélectifs : EP 0 570 920, WO 94-12466 qui décrit l'anandamide, US 4 371 720 qui décrit le CP 55940.

Par ailleurs, le récepteur CB₂ n'étant connu que depuis 1993, les nombreux brevets concernant des composés cannabinoïdes ne donnent aucune indication sur leur sélectivité. Parmi ces brevets, on peut citer notamment les brevets US 5 081 122, US 5 292 736, US 5 013 837, EP 0 444 451 qui décrivent des composés ayant une structure indolique ou indènique.

D'autres dérivés d'indole à activité cannabinoïde sont décrits dans J.W. Hufman et al., Biorg. Med. Chem. Lett. (1994), 4, 563.

On a maintenant trouvé de nouveaux composés agonistes spécifiques du récepteur CB₂ humain ayant une affinité élevée pour ledit récepteur, qui sont de puissants immuno-modulateurs et qui peuvent être utilisés sans risque de l'effet secondaire indiqué ci-dessus.

Dans la présente description, on désigne par "affinité élevée pour le récepteur CB₂ humain" une affinité caractérisée par une constante d'affinité inférieure ou égale à 10 nM et par "spécifique" les composés dont la constante d'affinité pour le récepteur CB₂ est au moins 30 fois inférieure à la constante d'affinité pour le récepteur CB₁ et pour lesquels la constante d'affinité pour le récepteur CB₁ est supérieure ou égale à 100 nM. De plus, la spécificité des composés de l'invention se manifeste également vis-à-vis d'autres récepteurs ; les composés de l'invention ont en effet une constante d'inhibition pour des récepteurs humains autres que les récepteurs aux cannabinoïdes supérieure à 1 µM.

Ainsi, la présente invention a pour obiet les composés de formule (Ib₂) : 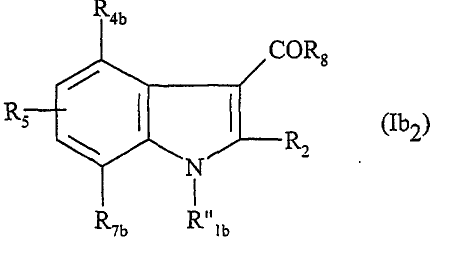 dans laquelle :
- R"_{1b} représente le groupe -(CH₂)ₙZ ;
- R_{4b} représente l'hydrogène, un (C₁-C₄)alkyle, un (C₁-C₄)alcoxy, un atome d'halogène, un groupe -CF₃, un groupe -OCF₃ ou un (C₁-C₄)alkylthio ;
- R_{7b} représente l'hydrogène, un (C₁-C₄)alkyle, un (C₁-C₄)alcoxy, un atome d'halogène, un groupe -CF₃, un groupe -OCF₃ ou un (C₁-C₄)alkylthio ;
- R₂ représente l'hydrogène, un halogène ou un (C₁-C₄)alkyle ;
- R₅ représente l'hydrogène, un (C₁-C₄)alkyle, un (C₁-C₄)alcoxy, un atome d'halogène, un groupe -CF₃, un groupe -OCF₃, un (C₁-C₄)alkylthio ;
- R₈ représente un phényle substitué une à quatre fois par un substituant choisi parmi : un halogène, un (C₁-C₄)alkyle et un (C₁-C₄)alcoxy ; un polycycle choisi parmi un napht-1-yle, un napht-2-yle, un 1,2,3,4-tétrahydronapht-l-yle, un 1,2,3,4-tétrahydronapht-5-yle, un anthryle, un benzofuryle, un benzothièn-2-yle, un benzothièn-3-yle, un 2-, 3-, 4- ou 8-quinolyle, lesdits polycycles étant non substitués ou substitués une ou deux fois par un substituant choisi parmi : un (C₁-C₄)alkyle, un (C₁-C₄)alcoxy, un (C₁-C₄)alkylthio, un halogène, un cyano, un hydroxyle, un trifluorométhyle, et un imidazol-1-yle ;
- n est 2, 3, 4 ou 5 ;
- Z représente le groupe méthyle ou un atome d'halogène, et leurs sels pharmaceutiquement acceptables, à condition que :
- au moins l'un des groupes R_{4b}, R₅ et R_{7b} représente l'hydrogène ;
- lorsque Z est le brome, n est 3 ou 4, R_{4b}, R₅ et R_{7b} sont l'hydrogène et R₂ est un groupe méthyle alors R₈ est différent des groupes 1-naphtyle et 4-méthoxyphényle ;
- lorsque R₈ est un groupe 4-méthoxyphényle, R₂ est un groupe méthyle, R_{4b} et R_{7b} sont l'hydrogène, R₅ est un atome de fluor en position 5 et n est 3 alors Z est différent des atomes de brome et chlore ;
- lorsque Z est le groupe méthyle, R_{4b}, R₅ et R_{7b} sont l'hydrogène et R₂ est le groupe méthyle alors R₈ est différent du groupe 1-naphtyle. lorsque Z est le brome, n est 3 ou 4, R_{4b}, R₅ et R_{7b} sont l'hydrogène et R₂ est un groupe méthyle alors R₈ est différent des groupes 1-napthyle et 4-méthoxyphényle.

Les composés de formule (Ib₂) selon l'invention peuvent être obtenus par différents procédés de synthèse faisant appel notamment à des étapes d'acylation et de cyclisation bien connues de l'homme du métier.

Les composés de formule (Ib₂) peuvent être obtenus par le procédé B/ représenté sur le schéma III ci-après.

Ce procédé, qui est décrit notamment dans le brevet US 4 581 354, consiste :
1/ à faire réagir un dérivé d'indole de formule (5), convenablement substitué en position 2 par un groupe R₂ tel que défini précédemment avec un halogénure de formule X(CH₂)ₙZ dans laquelle, n et Z sont tels que définis précédemment et X représente un atome d'halogène, par exemple le chlore, le brome ou l'iode, pour former un composé de formule (6) dans laquelle R"_{1b} est le groupe (CH₂)ₙZ ;
2/ à soumettre le composé de formule (6) ainsi obtenu à une acylation avec un halogénure d'acide de formule R₈-CO-X dans laquelle X est un halogène, par exemple le chlore ou le brome et R₈ est tel que défini précédemment pour former un composé de formule (Ib₂).
3/ éventuellement à transformer le composé de formule (Ib₂) ainsi obtenu en l'un de ses sels pharmaceutiquement acceptables.

L'étape 1/ du procédé B est avantageusement réalisée en présence d'une base dans un solvant organique inerte dans les conditions de réaction.

A titre d'exemple de base, on peut utiliser un carbonate de métal alcalin, tel que le carbonate de sodium ou le carbonate de potassium, un hydrure, tel que l'hydrure de sodium ou un hydroxyde de métal alcalin, tel que l'hydroxyde de potassium. On préfère utiliser tout particulièrement l'hydroxyde de potassium.

Comme solvants, on peut utiliser par exemple le toluène, le diméthylformamide (DMF) ou le diméthylsulfoxyde (DMSO), ce dernier étant préféré. La réaction est réalisée entre 0° C et la température d'ébullition du solvant.

De façon, particulière, on peut également effectuer la réaction en présence de tris[2-(2-méthoxyéthoxy)éthyl]amine (TDA-1).

L'étape 2/ du procédé B est une acylation par réaction de Friedel et Crafts, réalisée en présence d'un acide de Lewis tel que le chlorure d'aluminium dans un solvant inerte tel que le dichloro-1,2-éthane ou le disulfure de carbone. La réaction d'acylation peut également s'effectuer en présence d'un acide de Lewis tel que le dichlorure d'éthylmagnésium dans un solvant inerte tel que le dichlorométhane selon le procédé décrit dans J. Med. Chem., 1995, 38, 3094.

Selon une variante de l'étape 1/ du procédé B (procédé B') on peut également préparer le composé de formule (6) en suivant les trois étapes représentées sur le schéma III' ci-après. Cette variante consiste à faire réagir une aniline de formule (9) avec une cétone de formule RSCH₂COR₂ dans laquelle R représente un méthyle ou un phényle, en présence d'hypochlorite de *tert*-butyle (tBuOCl) selon le procédé décrit dans J. Am. Chem. Soc., 1974,96, 5495. Le composé (10) ainsi obtenu est ensuite alkylé dans des conditions analogues à celles décrites ci-dessus pour l'étape 1/ du procédé B pour obtenir le composé de formule (11). On élimine ensuite le groupe sulfuré par réaction avec le nickel de Raney ou par réaction avec l'acide 2-mercaptobenzoïque dans l'acide trifluoroacétique selon le procédé décrit dans Tetrahedron Lett., 1993, 34 (13), 2059-2062 pour obtenir le composé de formule (6).

Selon une variante également décrite dans le brevet US 4 581 354 (Procédé B₁ représenté sur la Schéma IV) on procède d'abord à une acylation du composé (5) par réaction avec un halogénure de méthylmagnésium et un halogénure d'acide de formule R₈COX dans l'éther pour former le composé (7), puis on procède à l'addition du substituant R"_{1b} par réaction du composé (7) avec un halogénure XR"_{1b} en présence d'une base dans des conditions analogues à celles décrites ci-dessus pour l'étape 1/ du procédé B.

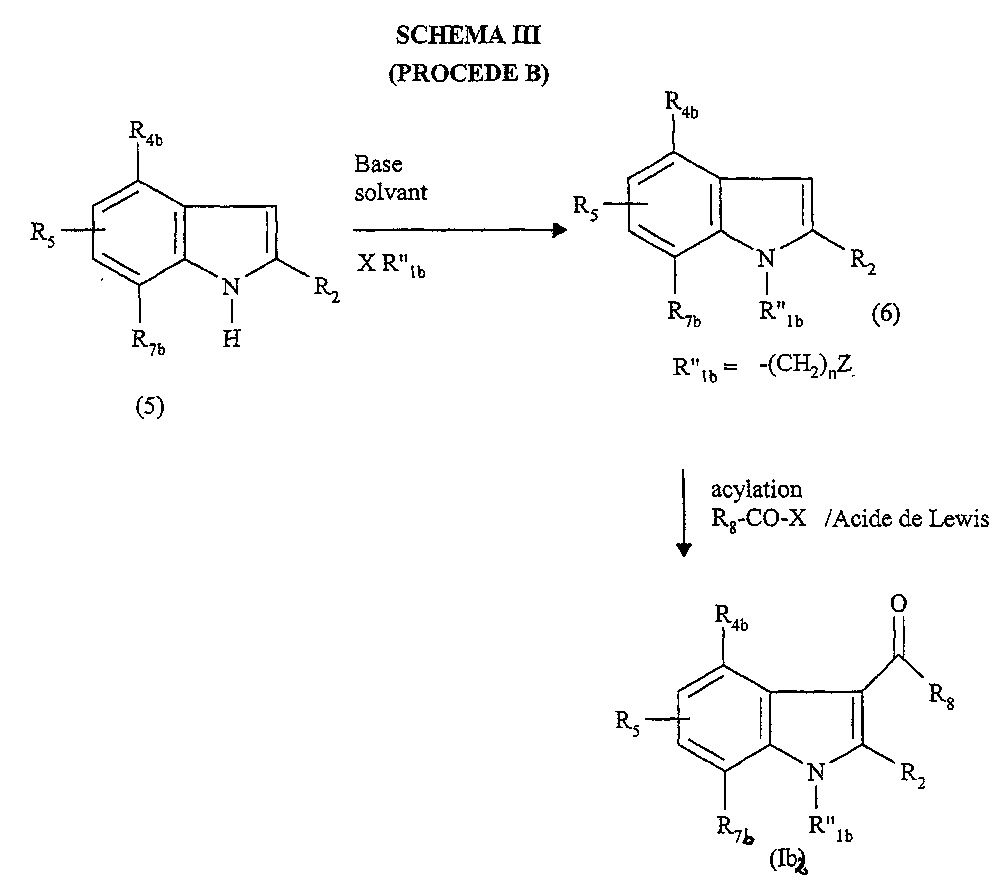

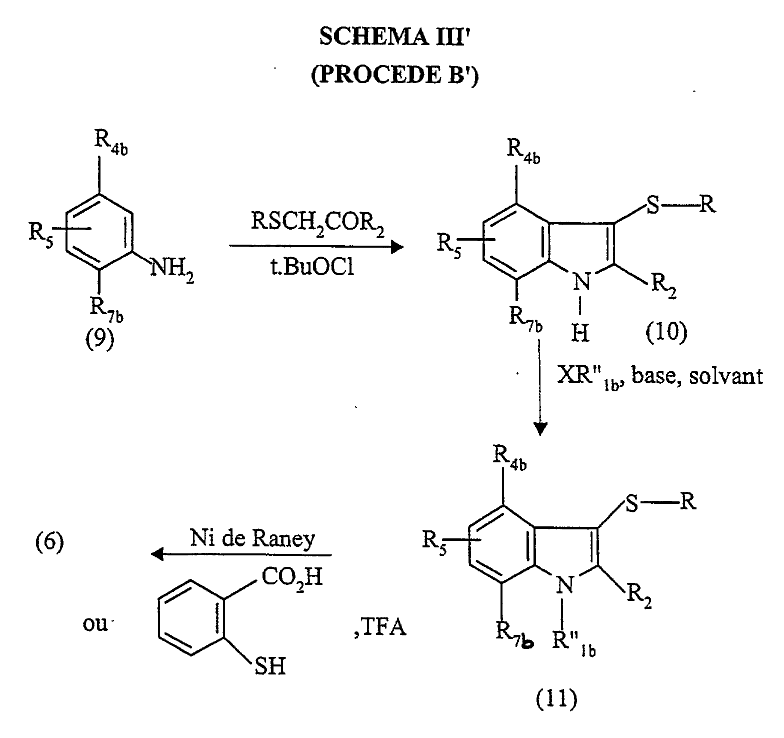

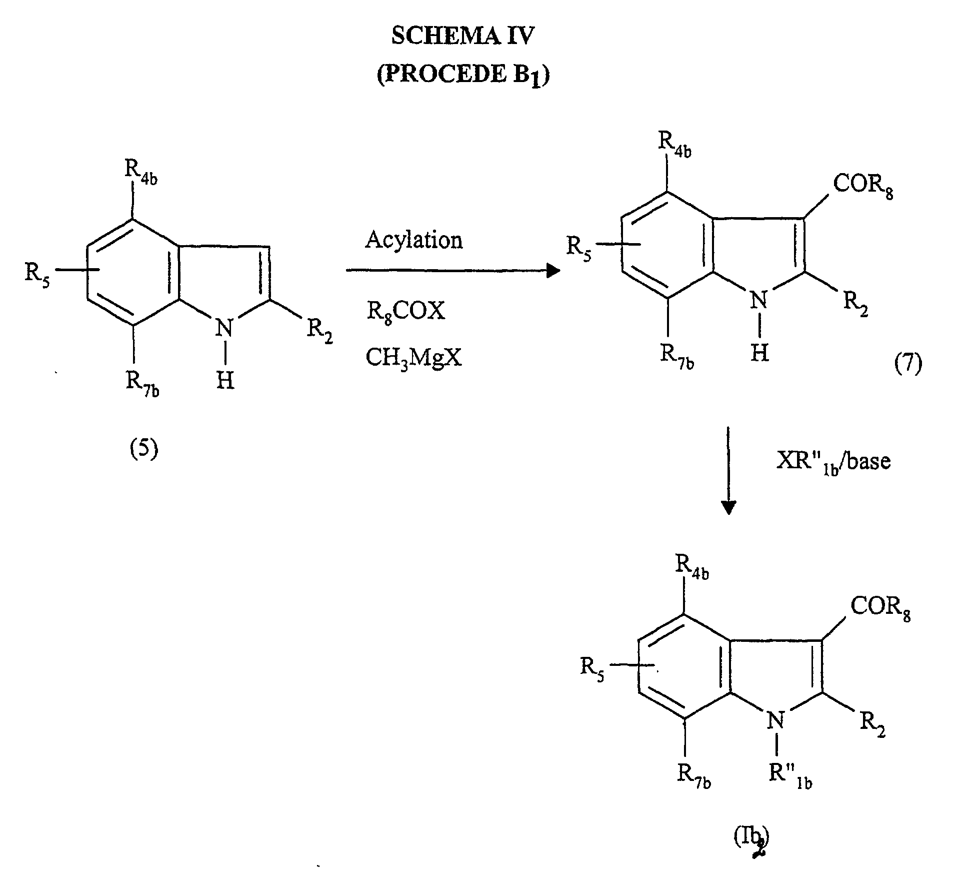

Parmi ces composés, ceux qui sont particulièrement préférés sont les composés ci-après :
* le 1-n-pentyl-2-méthyl-3-(4-chloro-1-naphtylcarbonyl)-7-méthoxyindole ;
* le 1-n-pentyl-2-méthyl-3-(4-bromo-1-naphtylcarbonyl)-7-méthoxyindole ;
* le 1-n-pentyl-2-méthyl-3-(4-bromo-1-naphtylcarbonyl)-7-fluoroindole ;

Les composés selon l'invention sont généralement administrés en unités de dosage.

Lesdites unités de dosage sont de préférence formulées dans des compositions pharmaceutiques dans lesquelles le principe actif est mélangé avec un excipient pharmaceutique.

Selon un autre de ses aspects, la présente invention concerne des compositions pharmaceutiques renfermant, en tant que principe actif, un composé de formule (Ib₂) ayant une affinité élevée pour le récepteur CB₂ humain, caractérisée par une constante d'inhibition Ki inférieure ou égale à 10 nM dans les études de fixation du ligand.

Les composés de formule (Ib₂) et leurs sels pharmaceutiquement acceptables peuvent être utilisés à des doses journalières de 0,1 à 100 mg par kilo de poids corporel du mammifère à traiter, préférentiellement à des doses journalières de 0,2 à 50 mg/kg. Chez l'être humain, la dose peut varier de préférence de 0,5 à 1 000 mg par jour, plus particulièrement de 1 à 500 mg selon l'âge du sujet à traiter ou le type de traitement : prophylactique ou curatif.

Les maladies pour le traitement desquelles les composés et leurs sels pharmaceutiquement acceptables peuvent être utilisés, sont par exemple les maladies autoimmunes, les maladies infectieuses, les maladies allergiques. Plus particulièrement on peut citer, les maladies autoimmunes suivantes : lupus érythémateux disséminé, les maladies du tissu conjonctif ou connectivites, le syndrome de Sjôgren's, la spondylarthrite ankylosante, l'arthrite réactive, la spondylarthrite indifférenciée, la maladie de Behcet's, les anémies autoimmunes hémolytiques. Les maladies allergiques à traiter peuvent être du type hypersensibilité immédiate ou asthme, par exemple. De même les composés et leurs sels pharmaceutiquement acceptables peuvent être utilisés pour traiter les vascularites, les infections parasitaires, l'amylose, les maladies affectant la lignée plasmocytaire.

Dans les compositions pharmaceutiques de la présente invention pour l'administration orale, sublinguale, sous-cutanée, intramusculaire, intraveineuse, transdermique, locale ou rectale, ou par inhalation, les principes actifs peuvent être administrés sous formes unitaires d'administration, en mélange avec des supports pharmaceutiques classiques, aux animaux et aux êtres humains. Les formes unitaires d'administration appropriées peuvent être choisies en fonction des maladies à traiter ; elles comprennent les formes par voie orale, telles que les comprimés, les gélules, les poudres, les granules et les solutions ou suspensions orales, les formes d'administration par inhalation, les formes d'administration sublinguale et buccale, les formes d'administration sous-cutanée, intramusculaire, intraveineuse, intranasale ou intraoculaire et les formes d'administration rectale. Les formes d'administration par voie orale, intraveineuse ou par inhalation sont préférées.

Lorsque l'on prépare une composition solide sous forme de comprimés, on mélange le principe actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrolidone, de même qu'avec des édulcorants ou des correcteurs du goût.

Pour une administration, rectale, on recourt à des suppositoires qui sont préparés avec des liants fondant à la température rectale, par exemple du bourre de cacao ou des polyélhylèneglycols.

Pour une administration parentérale, intranasale ou intraoculaire, on utilise des suspensions aqueuses, des solutions salines isotoniques ou des solutions injectables qui contiennent des agents de dispersion et/ou des agents mouillants pharmacologiquement compatibles, par exemple le propylèneglycol ou le butylèneglycol.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible.

Le principe actif peut être formulé également sous forme de microcapsules, éventuellement avec un ou plusieurs supports ou additifs.

Dans chaque unité de dosage le principe actif de formule (Ib₂) est présent dans les quantités adaptées aux doses journalières envisagées, En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,5 à 1 000 mg de principe actif, avantageusement de 1 à 500 mg, de préférence de 1 à 200 mg devant être administrés une à quatre fois par jour.

Les compositions susdites peuvent également renfermer d'autres produits actifs utiles pour la thérapeutique souhaitée tels que, par exemple, des corticostéroïdes et des β2 agonistes.

Grâce à leur très forte affinité pour le récepteur CB₂ humain et à leur grande sélectivité, les composés selon l'invention pourront être utilisés, sous forme radiomarquée comme réactifs de laboratoire.

Par exemple, ils permettent d'effectuer la caractérisation, l'identification et la localisation du récepteur CB₂ humain dans des coupes de tissus, ou du récepteur CB₂ chez l'animal entier par autoradiographie.

Les composés selon l'invention permettent également d'effectuer le tri ou screening des molécules en fonction de leur affinité pour le récepteur CB₂ humain. On opère alors par une réaction de déplacement du ligand radiomarqué, objet de la présente invention, de son récepteur CB₂ humain.

Des exemples de composés appropriés aux fins de l'invention sont les composés décrits dans les exemples 1 à 3 ci-après.

Dans les exemples qui vont suivre, les abréviations suivantes sont utilisées :
TA : température ambiante
F : point de fusion
DCM : dichlorométhane
THF : tétrahydrofurane
DMF : diméthylformamide
DMSO : diméthylsulfoxyde
AcOEt : acétate d'éthyle
iPr : isopropyle
HCl : acide chlorhydrique
TFA : acide trifluoroacétique
NaCl : chlorure de sodium
NaH : hydrure de sodium
KOH : potasse
TDA-1 : tris[2-(2-méthoxyéthoxy)éthyl]amine
MgSO₄ : sulfate de magnésium
NaOH : soude
NH₄Cl : chlorure d'ammonium
Na₂CO₃ : carbonate de sodium.
s : singulet
t : triplet
m : multiplet

### EXEMPLE 1 :

### 1 -n-Pentyl-2-méthyl-3-(4-chloro-1-naphtylcarbonyl)-7-méthoxyindole :

### A/ 2-Méthyl-3-(4-chloro-1-naphtylcarbonyl)-7-méthoxyindole :

On ajoute goutte à goutte une solution de 1,02 g de 2-méthyl-7-méthoxyindole dans 5 ml d'éther à une solution de 2,60 ml de bromure de méthylmagnésium 3,0 M dans l'éther. On dilue dans 6 ml d'éther et on refroidit à 0°C.

Le mélange est agité pendant 1 heure à température ambiante puis refroidi à 0°C. On ajoute goutte à goutte une suspension de chlorure d'acide 4-chloro 1-naphtoïque dans une solution composée de 6 ml d'éther et de 4 ml de THF au mélange obtenu précédemment.

Le mélange est ensuite agité pendant 16 heures à température ambiante puis 2 heures à reflux.

On procède après à une hydrolyse par 50 ml d'eau glacée à laquelle on a ajouté 50 ml d'une solution saturée de NH₄Cl.

Les solvants sont évaporés sous vide et la phase aqueuse est extraite au DCM puis lavée à l'eau. On sèche sur du MgSO₄.

Les solvants sont évaporés puis on purifie le produit obtenu par chromatographie sur gel de silice en utilisant comme éluant du CH₂Cl₂.

On obtient le produit du titre (F = 184°C).

### B/ 1-n-pentyl-2-méthyl-3-(4-chloro-1-naphtylcarbonyl)-7-methoxyindole.

On ajoute 0,13 g de NaH (à 60 % dans l'huile) à une solution de 0,77 g de l'indole préparé à l'étape A/ dans 10 ml de DMF.

On agite pendant 10 min. puis on ajoute 0,43 ml de 1-iodopentane et on chauffe le mélange à 100°C pendant 16 heures.

Le mélange est ensuite versé dans 100 ml d'une solution saturée de NH₄Cl à 0°C et extrait à l'acétate d'éthyle.

La phase organique est lavée à l'eau, séchée sur MgSO₄ puis purifiée par chromatographie sur gel de silice (éluant = toluène). Le produit cristallise dans un mélange DCM/iPr₂O, F = 112°C.

On obtient 0,34 g du produit du titre. (F = 112°C).

### EXEMPLE 2

### 1-n-Pentyl-2-méthyl-3-(4-bromo-1-naphtylcarbonyl)-7-méthoxyindole.

### A) 2-Méthyl-3-(4-bromo-1-naphtylcarbonyl)-7-méthoxyindole.

On refroidit à 0°C une solution de 14,7 ml de bromure de méthylmagnésium 3M dans l'éther diluée dans 30 ml d'éther et ajoute goutte à goutte une solution de 5,6 g de 2-méthyl-7-méthoxyindole dans 25 ml d'éther puis on laisse 1 heure sous agitation à TA.

On ajoute ensuite une solution de 12,3 g de chlorure de l'acide 4-bromo-1-naphtoïque dans 28 ml d'éther et 19 ml de THF, chauffe à reflux pendant 1 heure et laisse 16 heures sous agitation à TA. On verse le mélange réactionnel sur 350 ml d'eau glacée, ajoute 40 g de NH₄Cl et concentre sous vide les solvants. On extrait la phase aqueuse à l'éther, lave la phase organique par 50 ml d'une solution saturée de NH₄Cl, par 100 ml d'une solution saturée de NaCl, sèche sur MgSO₄ et évapore sous vide le solvant. On chromatographie le résidu sur gel de silice en éluant au DCM. On obtient 2 g du produit attendu après recristallisation dans le toluène.

### B) 1-n-pentyl)-2-méthyl-3-(4-bromo-1-naphtylcarbonyl)-7-méthoxyindole.

On chauffe à 90°C pendant 2 heures un mélange de 0,46 g du composé obtenu à l'étape A, 0,49 g de 1-iodopentane, 0,16 g de TDA-1 et 0,16 g de KOH broyée dans 7 ml de toluène. On verse le mélange réactionnel dans 20 ml d'eau, après décantation on lave la phase organique par une solution d'HCl à 10 %, à l'eau, par une solution saturée de NaCl, sèche sur MgSO₄ et évapore sous vide le solvant. On chromatographie le résidu sur gel de silice en éluant par le mélange AcOEt/toluène (50/50 ; v/v). On obtient 0,5 g du produit attendu (F = 114,5°C).

### EXEMPLE 3

### 1-n-Pentyl-2-méthyl-3-(4-bromo-1-naphtylcarbonyl)-7-fluoroindole.

### A) 2-Méthyl-3-(méthylthio)-7-fluoroindole.

On refroidit à -65°C sous atmosphère d'azote une solution de 20 g 2-fluoroaniline dans 600 ml de DCM, ajoute goutte à goutte une solution de 23,4 g d'hypochlorite de *tert*-butyle dans 30 ml de DCM et laisse 10 minutes sous agitation. On ajoute ensuite à -65°C une solution de 22,5 g de (méthylthio)acétone dans 30 ml de DCM et laisse 2 heures sous agitation à -65°C. On laisse remonter la température à -40°C, ajoute une solution de 30 ml de triéthylamine dans 30 ml de DCM et laisse sous agitation en laissant remonter la température à TA. On hydrolyse par ajout de 200 ml d'eau, après décantation lave la phase organique à l'eau, par une solution saturée de NaCI, sèche sur MgSO₄ et évapore sous vide le solvant. On chromatographie le résidu sur gel de silice en éluant au toluène. On obtient 20,5 g du produit attendu.

### B) 2-Méthyl-7-fluoroindole.

A une solution de 10 g du composé obtenu à l'étape précédente dans 100 ml de TFA on ajoute à TA 16 g d'acide 2-mercaptobenzoïque et laisse 2 heures sous agitation à TA. On filtre l'insoluble et concentre sous vide le filtrat. On reprend le résidu par 100 ml du mélange AcOEt/eau (50/50 ; v/v), lave la phase organique trois fois par une solution à 10 % de NaOH, à l'eau, par une solution à 10 % d'HCl, par une solution saturée de NaCl, sèche sur MgSO₄ et évapore sous vide le solvant. On chromatographie le résidu sur gel de silice en éluant au toluène. On obtient 3,8 g du produit attendu.

### C) 1-n-Pentyl-2-méthyl-7-fluoroindole.

On chauffe à 95°C pendant 5 heures un mélange de 1 g du composé obtenu à l'étape B, 1,6 g de 1-iodopentane, 0,21 g de TDA-1 et 0,75 g de KOH finement broyée dans 15 ml de toluène. Après refroidissement à TA, on ajoute 30 ml d'eau, décante, lave la phase organique par une solution à 10 % d'HCl, à l'eau, par une solution saturée de NaCl, sèche sur MgSO₄ et évapore sous vide le solvant. On chromatographie le résidu sur gel de silice en éluant par le mélange cyclohexane/toluène (60/40 ; v/v). On obtient 0,58 g du produit attendu.

### D) 1-n-Pentyl-2-méthyl-3-(4-bromo-1-naphtylcarbonyl)-7-fluoroindole.

On refroidit à 0°C sous atmosphère d'azote une solution de 0,58 g du composé obtenu à l'étape précédente et 0,95 g du chlorure de l'acide 4-bromo- 1-naphtoïque dans 25 ml de DCM, ajoute goutte à goutte 3,17 ml d'une solution 1,8 M de dichlorure d'éthylaluminium dans le toluène et laisse 24 heures sous agitation à TA. On verse le mélange réactionnel dans 100 ml d'eau glacée, extrait au DCM, lave la phase organique par une solution à 5 % de Na₂CO₃, à l'eau, par une solution saturée de NaCl, sèche sur MgSO₄ et évapore sous vide le solvant. On chromatographie le résidu sur gel de silice en éluant par le mélange AcOEt/toluène (50/50 ; v/v). On obtient 0,27 g du produit attendu.
RMN (200 MHz) ; DMSO (2,5 ppm) ; DOH (3,3 ppm) ; 0,85 ppm : t : 3H ; 1,3 ppm : m : 4H ; 1,75 ppm : m : 2H ; 2,35 ppm : s : 3H ; 4,3 ppm : t : 2H ; 6,8-8,4 : m : 9H.

### Tests biochimiques

On a montré qu'à des concentrations nanomolaires, des composés tels que le méthane sulfonate de 3-(4-morpholinyl méthyl)-5-méthyl-6-(1-naphtylcarbonyl)-2,3-dihydropyrrolo[1,2,3-de]-1,4-benzoxazine et le 1-(2-(4-morpholinyl)éthyl)-2-méthyl-3-(1-naphtylcarbonyl)-7-méthoxyindole sont capables d'augmenter sensiblement le taux de synthèse d'ADN de cellules B humaines costimulées avec des anticorps anti-Ig (augmentation d'environ 40 % de l'absorption de la thymidine).

Lorsque l'on a utilisé l'antagoniste sélectif du récepteur CB₁ (SR 141716A) dans un large domaine de concentration en même temps que le composé CP 55940 (ou Δ⁹-THC ou WIN 55212-2) à 10⁻⁹ M, on n'a observé aucun effet de blocage.

On a pu observer le même phénomène, c'est-à-dire une augmentation de la croissance des cellules B, en utilisant une autre voie d'activation, consistant à stimuler les cellules B humaines par mise en contact de l'antigène CD 40 avec des anticorps monoclonaux présentés par des cellules L CD W32.

Les composés selon l'invention (Ib₂) et leurs sels éventuels ont montré une affinité *in vitro* de 30 à 1 000 fois supérieure pour les récepteurs aux cannabinoïdes humains périphériques (CB₂) que pour les récepteurs humains centraux (CB₁), exprimés dans des cellules ovariennes de hamster chinois (CHO). Les essais de liaison par affinité (binding) ont été réalisés selon les conditions expérimentales décrites par Devane et al. (Molecular Pharmacology, (1988), 34, 605-613), avec des membranes issues de lignées cellulaires dans lesquelles les récepteurs CB₁ et CB₂ ont été exprimés (Munro et al. Nature, (1993), 365; 561-565).

Les composés préférés sont les composés suivants :
* 1-n-pentyl-2-méthyl-3-(4-bromo-1-naphtylcarbonyl)-7-méthoxyindole ;
* 1-n-pentyl-2-méthyl-3-(4-bromo-1-naphtylcarbonyl)-7-fluoroindole.

D'autre part les composés selon l'invention se comportent *in vitro* comme des agonistes spécifiques des récepteurs humains aux cannabinoïdes CB₂ versus CB₁, exprimés dans des cellules CHO. En effet, en se liant spécifiquement aux récepteurs CB₂, ils diminuent la production d'AMPc stimulée par de la forskoline et ce en inhibant l'adénylate cyclase. Les essais ont été réalisés selon les conditions expérimentales décrites par Matsuda et al. (Nature. 1990, 346. 561-564).

Les composés selon l'invention possèdent également une affinité *in vivo* pour les récepteurs aux cannabinoïdes présents au niveau de la rate de souris lorsqu'ils sont administrés par voie intraveineuse, intrapéritonéale ou orale. Les essais ont été réalisés selon les conditions expérimentales décrites par Rinaldi-Carmona et al., (Life Sciences, 1995, 56, 1941-1947).

## Revendications

1. Composés de formule (Ib₂) : 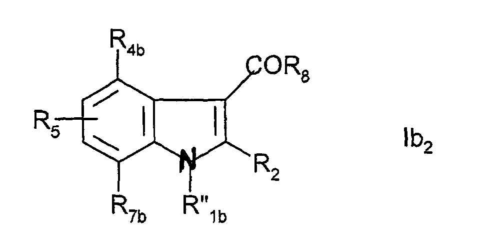 dans laquelle :
- R"_{1b} représente le groupe -(CH₂)ₙZ ;
- R_{4b} représente l'hydrogène, un (C₁-C₄)alkyle, un (C₁-C₄)alcoxy, un atome d'halogène, un groupe -CF₃, un groupe -OCF₃ ou un (C₁-C₄)alkylthio ;
- R_{7b} représente l'hydrogène, un (C₁-C₄)alkyle, un (C₁-C₄)alcoxy, un atome d'halogène, un groupe -CF₃, un groupe -OCF₃ ou un (C₁-C₄)alkylthio ;
- R₂ représente l'hydrogène, un halogène ou un (C₁-C₄)alkyle;
- R₅ représente l'hydrogène, un (C₁-C₄)alkyle, un (C₁-C₄)alcoxy, un atome d'halogène, un groupe CF₃, un groupe OCF₃, un (C₁-C₄)alkylthio;
- R₈ représente un phényle substitué une à quatre fois par un substituant choisi parmi : un halogène, un (C₁-C₄)alkyle, un (C₁-C₄)alcoxy ; un polycycle choisi parmi un napht-1-yle, un napht-2-yle, un 1,2,3,4-tétrahydronapht-1-yle, un 1,2,3,4-tétrahydronapht-5-yle, un anthryle, un benzofuryle, un benzothièn-2-yle, un benzothièn-3-yle, un 2-,3-,4-, ou 8-quinolyle, lesdits polycycles étant non substitués ou substitués une ou deux fois par un substituant choisi parmi : un (C₁-C₄)alkyle, un (C₁-C₄)alcoxy, un (C₁-C₄)alkylthio, un halogène, un cyano, un hydroxyle, un trifluorométhyle, et un imidazol-1-yle ;
- n est 2, 3 , 4 ou 5 ;
- Z représente le groupe méthyle ou un atome d'halogène ; et leurs sels pharmaceutiquement acceptables, à condition que :
- au moins l'un des groupes R_{4b}, R₅ et R_{7b} représente l'hydrogène ;
- lorsque Z est le brome, n est 3 ou 4, R_{4b}, R₅, et R_{7b} sont l'hydrogène et R₂ est un groupe méthyle alors R₈ est différent des groupes 1-naphtyle et 4-méthoxyphényle ;
- lorsque R₈ est un groupe 4-méthoxyphényle, R₂ est un groupe méthyle, R_{4b} et R_{7b} sont l'hydrogène, R₅ est un atome de fluor en position 5 et n est 3 alors Z est différent des atomes de brome et de chore;
- lorsque Z est le groupe méthyle, R_{4b}, R₅, et R_{7b} sont l'hydrogène et R₂ est le groupe méthyle alors R₈ est différent du groupe 1-naphtyle.

2. Composé selon la revendication 1, **caractérisé en ce qu'**il est le
* le 1-n-pentyl-2-méthyl-3-(4-chloro-1-naphtylcarbonyl)-7-méthoxyindole ;

3. Composé selon la revendication 1, **caractérisé en ce qu'**il est l'un des composés ci-après :
* le 1-n-pentyl-2-méthyl-3-(4-bromo-1-naphtylcarbonyl)-7-méthoxyindole ;
* le 1-n-pentyl-2-méthyl-3-(4-bromo-1-naphtylcarbonyl)-7-fluorindole ;

4. Composition pharmaceutique contenant à titre de principe actif, un composé selon l'une des revendications 1 à 3, ou un de ses sels pharmaceutiquement acceptables.

5. Utilisation des composés selon l'une quelconque des revendications 1 à 3 pour la préparation de médicaments immunomodulateurs.

## Claims

1. Compounds of formula (Ib₂) : 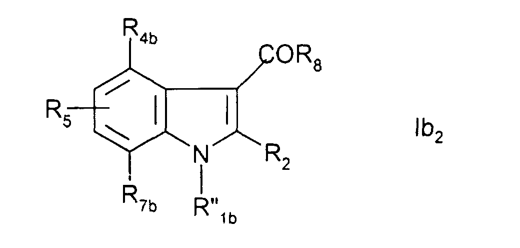 in which :
- R"_{1b} is the group -(CH₂)ₙZ ;
- R_{4b} is hydrogen, a (C₁-C₄)alkyl, a (C₁-C₄)alcoxy, a halogen atom, a group -CF₃, a group -OCF₃ or a (C₁-C₄)alkylthio;
- R_{7b} is hydrogen, a (C₁-C₄)alkyl, a (C₁-C₄)alkoxy, a halogen atom, a group -CF₃, a group -OCF₃ or a (C₁-C₄)alkylthio;
- R₂ is hydrogen, a halogen or a (C₁-C₄)alkyl;
- R₅ is hydrogen, a (C₁-C₄)alkyl, a (C₁-C₄)alkoxy, a halogen atom, a group CF₃, a group OCF₃ or a (C₁-C₄)alkylthio;
- R₈ is a phenyl monosubstituted to tetrasubstituted by a substituent selected from a halogen, a (C₁-C₄)alkyl and a (C₁-C₄)alkoxy; or a polycyclic radical selected from a naphth-1-yl, a naphth-2-yl, a 1,2,3,4-tetrahydronaphth-1-yl, a 1,2,3,4-tetrahydronaphth-5-yl, an anthryl, a benzofuryl, a benzothien-2-yl, a benzothien-3-yl and a 2-, 3-, 4- or 8-quinolyl, said polycyclic radicals being unsubstituted or monosubstituted or disubstituted by a substituent selected from a (C₁-C₄)alkyl, a (C₁-C₄)alkoxy, a (C₁-C₄)alkylthio, a halogen, a cyano, a hydroxyl, a trifluoromethyl and an imidazol-1-yl;
- n is 2, 3, 4 or 5;
- Z is the methyl group or a halogen atom; and
and their pharmaceutically acceptable salts,
with the proviso that
- at least one of the group R_{4b}, R₅ and R_{7b} is hydrogen,
- when Z is bromine, n is 3 or 4, R_{4b}, R₅ and R_{7b} are hydrogen and R₂ is a methyl group, then R₈ is different from naphth-1-yl and 4-methoxyphenyl groups;
- when R₈ is a 4-methoxyphenyl group, R₂ is a methyl group, R_{4b} and R_{7b} are hydrogen, R₅ is a fluor atom in position 5 and n is 3, then Z is different from chlor or fluor atoms;
- when Z is methyl group, R_{4b}, R₅ and R_{7b} are hydrogen and R₂ is a methyl group, then R₈ is different from naphtyl-1-yl group ;

2. Compound according to claim 1, **characterized in that** it is the 1-n-pentyl-2-methyl-3-(4-chloro-1-naphtylcarbonyl)-7-methoxyindole;

3. Compound according to claim 1, **characterized in that** it is one of the following compounds :
1-n-pentyl-2-methyl-3-(4-bromo-1-naphtylcarbonyl)-7-methoxyindole ;
1-n-pentyl-2-methyl-3-(4-bromo-1-naphtylcarbonyl)-7-fluorindole ;

4. Pharmaceutical composition containing as an active principle a compound according to any one of claims 1 to 3, or one of its pharmaceutically acceptable salts;

5. Use of the compounds according to anyone of claims 1 to 3 for the preparation of immunoindulating drugs.

## Patentansprüche

1. Verbindungen der Formel (Ib₂): 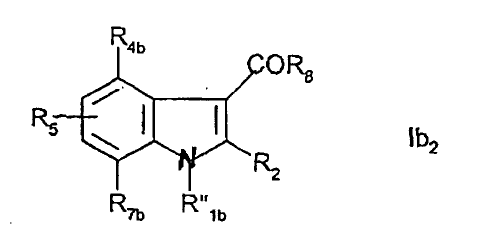 worin:
- R"_{1b} die Gruppe -(CH₂)ₙZ darstellt;
- R_{4b} Wasserstoff, (C₁-C₄)-Alkyl, (C₁-C₄)-Alkoxy, ein Halogenatom, eine Gruppe -CF₃, eine Gruppe -OCF₃ oder (C₁-C₄)-Alkylthio darstellt;
- R_{7b} Wasserstoff, (C₁-C₄)-Alkyl, (C₁-C₄)-Alkoxy, ein Halogenatom, eine Gruppe -CF₃, eine Gruppe -OCF₃ oder (C₁-C₄)-Alkylthio darstellt;
- R2 Wasserstoff, ein Halogen oder (C₁-C₄)-Alkyl darstellt,
- R₅ Wasserstoff, (C₁-C₄)-Alkyl, (C₁-C₄)-Alkoxy, ein Halogenatom, eine Gruppe -CF₃, eine Gruppe -OCF₃ oder (C₁-C₄)-Alkylthio darstellt;
- R₈ Phenyl darstellt, das ein- bis viermal substituiert ist, u.zw. durch einen Substituenten ausgewählt aus einem Halogen, (C₁-C₄)-Alkyl, (C₁-C₄)-Alkoxy; einem Polyzyklus ausgewählt aus Naphth-1-yl, Naphth-2-yl, 1,2,3,4-Tetrahydronaphth-1-yl, 1,2,3,4-Tetrahydronaphth-5-yl, einem Anthryl, einem Benzofuryl, einem Benzothien-2-yl, einem Benzothien-3-yl, einem 2-, 3-, 4- oder 8-Chinoyl, wobei die Polyzyklen gegebenenfalls ein- oder zweimal substituiert sind, u.zw. durch einen Substituenten ausgewählt aus (C₁-C₄)-Alkyl, (C₁-C₄)-Alkoxy, (C₁-C₄)-Alkylthio, einem Halogen, einem Cyano, einem Hydroxyl, einem Trifluormethyl und einem Imidazol-1-yl;
- n für 2, 3, 4 oder 5 steht;
- Z eine Methylgruppe oder ein Halogenatom darstellt;
sowie die pharmazeutisch annehmbaren Salze derselben, mit der Maßgabe, dass
- mindestens eine der Gruppen R_{4b}, R₅ und R_{7b} Wasserstoff darstellt;
- wenn Z Brom ist, n für 3 oder 4 steht, R_{4b}, R₅ und R_{7b} Wasserstoff darstellen und R₂ eine Methylgruppe ist, R₈ nicht 1-Naphthyl- oder 4-Methoxyphenyl ist;
- wenn R₈ 4-Methoxyphenyl ist, R₂ eine Methylgruppe darstellt, R_{4b} und R_{7b} Wasserstoff sind, R₅ ein Fluoratom in Position 5 ist und n für 3 steht, Z kein Brom- oder Chloratom ist;
- wenn Z eine Methylgruppe ist, R_{4b}, R₅ und R_{7b} Wasserstoff darstellen und R₂ eine Methylgruppe ist, R₈ nicht 1-Naphthyl ist.

2. Verbindung nach Anspruch 1, **dadurch gekennzeichnet, dass** sie
* 1-n-Pentyl-2-methyl-3-(4-chlor-1-naphthylcarbonyl)-7-methoxyindol ist.

3. Verbindung nach Anspruch 1, **dadurch gekennzeichnet, dass** sie eine der nachstehenden Verbindungen ist:
* 1-n-Pentyl-2-methyl-3-(4-brom-1-naphthylcarbonyl)-7-methoxyindol;
* 1-n-Pentyl-2-methyl-3-(4-brom-1-naphthylcarbonyl)-7-fluorindol.

4. Pharmazeutische Zusammensetzung, die als Wirkstoff eine Verbindung nach einem der Ansprüche 1 bis 3 oder ein pharmazeutisch annehmbares Salz derselben enthält.

5. Verwendung der Verbindungen nach einem der Ansprüche 1 bis 3 zur Herstellung von Immunmodulator-Medikamenten.
